# EUROPÄISCHE PATENTANMELDUNG

(11) **EP 1 721 684 A2**
(43) Veröffentlichungstag der Anmeldung: **15.11.2006**
(21) Anmeldenummer: 06009488.5
(22) Anmeldetag: 09.05.2006
(51) Int. Cl.: B08B 15/02, B01L 9/02, A61L 2/10, H01J 37/32

(54) **Nutzraum mit in-situ-Entkeimungsvorrichtung**

(30) Priorität: 09.05.2005 CH 8142005
(71) Anmelder: LICONIC AG, 9493 Mauren (LI)
(72) Erfinder: Löbach, Ernst, FL-9492 Eschen (LI)
(74) Vertreter: Blum, Rudolf Emil

(57) **Zusammenfassung**

Im Nutzraum (20) einer Einrichtung zur Aufbewahrung/Präparation von biologischen Proben ist eine erfindungsgemässe Quecksilberdampf-Hochdrucklampe (21) angeordnet. Mit einer derartigen Lampe kann in-situ auf effiziente Weise UV-Licht, Ozon und Wärme erzeugt werden, was es erlaubt, den Nutzraum (20), insbesondere Klimaschränke (48), sicher zu entkeimen.

## Beschreibung

Die Erfindung betrifft eine Einrichtung mit Nutzraum zur Aufbewahrung und/oder Präparation biologischer Proben und/oder Güter, insbesondere einen Klimaschrank sowie eine Quecksilberdampflampe und ein Verfahren zur Entkeimung von Nutzräumen.

Aus der klinischen Sterilisationstechnik sind verschiedene Verfahren zur Keimabtötung bekannt, insbesondere auch die Sterilisation mittels Heissluft oder Gasen wie Ethylenoxid und Formaldehyd. Im Allgemeinen treffen die jeweils eingesetzten klinischen Verfahren ein engeres Keimspektrum.

Ein typischer Klimaschrank besitzt einen Nutzraum zur Aufnahme biologischer Proben und kann als Brutschrank (Inkubator) oder Kühlschrank, z.B. in biologischen Laboratorien, verwendet werden. Er muss im Allgemeinen vor der Eingabe von Proben in einen definierten Anfangszustand (Nullzustand, "Reset") gesetzt werden, insbesondere muss der Nutzraum, einschliesslich ortsfester mechanischer Strukturen, frei sein von Keimen möglichst jeglicher Art.

Die Erfindung stellt sich die Aufgabe, diese Keimfreiheit für ein möglichst umfangreiches Spektrum aus der Vielfalt denkbarer Keime herzustellen. Diese Aufgabe wird von der Einrichtung gemäss Anspruch 1 gelöst.

Der Erfindung liegt der Gedanke zugrunde, verschiedene Verfahren gleichzeitig einzusetzen, um ein möglichst breites Keimspektrum anzusprechen, so wie es in der biologischen Laborpraxis vorkommt. Sie nutzt die Tatsache aus, dass sich in der Entkeimungsphase ausser den mechanischen Strukturen einschliesslich der Seitenwände keine Objekte, insbesondere keine biologischen Proben/Güter, im Nutzraum befinden. Daher können hier auch Verfahren eingesetzt werden, welche sonst in der Sterilisationstechnik nicht eingesetzt werden, nämlich Entkeimung durch Ozonbegasung sowie Bestrahlung mit sehr hartem ultraviolettem Licht (Wellenlängen ab 200nm).

Einerseits könnten nämlich die zur Sterilisierung eingegebenen Gegenstände selbst von Ozon und UV-Strahlung angegriffen werden, andererseits sind die abzutötenden Keime meist in den Gegenständen verborgen, so dass sowohl Ozon als auch UV-Strahlung keinen unmittelbaren Zugang zu den Keimen haben (Ozon zerfällt meist bei Vorkontakt mit Oberflächen, UV wird absorbiert, gegenseitiger Schattenwurf von eingegebenen Gegenständen).

Die erfindungsgemässe Einrichtung verwendet drei an sich bekannte Verfahren zur Keimabtötung gleichzeitig:
- Die UV-Bestrahlung
- Die Ozon-Begasung
- Die Heissluft-Zufuhr

Die keimtötende Wirkung von UV-Strahlung wird beschrieben in J. Kiefer, "Ultraviolette Strahlen", Walter de Gruyter, Berlin 1977. Die keimtötende Wirkung von Ozon wird beschrieben in M. Horvath, L. Bilitzky, J. Hüttner, "Ozone", Elsevier, Amsterdam 1985. Die Heissluftsterilisation ist allgemein bekannt.

Der Erfindung basiert auf der Idee, alle drei Verfahren mit einem einzigen Bauelement bereitzustellen und auf einfache Weise zu kombinieren, so dass relativ geringe Herstellungs- und Betriebskosten anfallen.

Dieses wesentliche Bauelement ist eine Quecksilberdampf-Hochdrucklampe, insbesondere in einer erfindungsgemässen speziellen Bauform. In Quecksilberdampf-Hochdrucklampen wird der grössere Teil des Lichts auf direktem oder indirektem Wege durch die Strahlung des Quecksilbers bei einem Partialdruck grösser als 100 Kilopascal erzeugt. Eine mögliche genormte Ausführungsform einer derartigen Quecksilberdampf-Hochdrucklampe ist in der Europäischen Norm EN 60188 beschrieben.

Quecksilberdampf-Hochdrucklampen sowie deren elektrische Versorgungsgeräte sind seit langem bekannt (Literatur: W. Elenbaas, "Quecksilberdampf-Hochdrucklampen", Philips Technische Bibliothek, Eindhoven 1966). Für die erfindungsgemässe Aufgabe jedoch wurden Quecksilberdampf-Hochdrucklampen bisher nie herangezogen.

Quecksilberdampf-Niederdrucklampen werden schon seit langem zur Sterilhaltung von Objekten in Behältnissen verwendet. Für die erfindungsgemässe Aufgabe sind diese Lampen jedoch im Allgemeinen zu schwach in Bezug auf Lichtintensität und Ozonerzeugung.

In DE 102 032 34 A1 wird ein Verfahren zur Dekontamination einer Sicherheitswerkbank ("Flow Box") vorgeschlagen, das Ozon von einem Ozongenerator ausserhalb des Arbeitsraumes in das Lüftungssystem der Sicherheitswerkbank einspeist und in den Arbeitsraum leitet. Als Ozongenerator wird wahlweise auch ein UV-Strahler vorgeschlagen (Anspruch 2 der vorerwähnten Anmeldung). Ein Verfahren mit dieser Anordnung hat jedoch erhebliche Nachteile.

Erstens zerfällt grundsätzlich ein Teil des erzeugten Ozons durch direkten Wandkontakt, insbesondere durch Kontakt mit den Wänden der Lüftungskanäle. Besonders nachteilig ist die Durchleitung durch Filter, da hier grosse Oberflächen und entsprechend häufige Wandkontakte vorliegen.

Zweitens besteht kein Sichtkontakt zwischen UV-Strahler und Arbeitsraum, so dass die unmittelbare, entkeimend wirkende UV-Strahlung nicht ausgenutzt werden kann.

Die vorgeschlagene Erfindung hat in einer vorteilhaften Ausführung diese Nachteile nicht, da die Ozon erzeugende Quecksilberdampf-Hochdrucklampe direkt im Nutzraum angebracht ist, so dass alle zur Entkeimung beitragenden, von der erfindungsgemässen Quecksilberdampf-Hochdrucklampe ausgehenden Wirkungen unmittelbar auf die Strukturen des Arbeits- bzw. Nutzraumes treffen.

In einem weiteren Aspekt betrifft die Erfindung die Verwendung einer Quecksilberdampflampe (nicht nur einer Quecksilberdampf-Hochdrucklampe) in einem Verfahren zur Sterilisation des Innenraums einer Einrichtung. Hierbei zeigt es sich, dass durch permanentes Zyklieren der Luft im Nutzraum infolge der von der Quecksilberdampflampe (21) ausgehenden ozonogenen UV-Strahlung die Ozonkonzentration bis zu bioziden Schwellwerten erhöht werden kann.

Ausserdem bezieht sich die Erfindung auf eine Quecksilberdampflampe bzw. Vorrichtung mit einer Quecksilberdampflampe, bei welcher ein das Licht durchlassendes Hüllrohr der Quecksilberdampflampe aus Quarzglas mit eingebetteten Nanopartikeln besteht, derart, dass das durch die Nanopartikel entstandene Blockfilter ozonogene Strahlung zwischen 180 nm und 230 nm durchlässt und ozonzerlegende Strahlung zwischen 230 nm und 280 nm unterdrückt.

Die Quecksilberdampflampe bzw. Qucksilberdampf-Hochdrucklampe kann in der Einrichtung bzw. dem Klimaschrank fest eingebaut sein. Sie kann jedoch auch nur bedarfsweise (d.h. bei einer gewünschten Entkeimung) in diese bzw. diesen eingesetzt werden.

Weitere bevorzugte Ausführungen und Vorteile der Erfindung ergeben sich aus der nun folgenden Beschreibung anhand der Figuren. Dabei zeigen
Fig. 1 eine Schnittansicht einer bevorzugten Ausführung der erfindungsgemässen Quecksilberdampf-Hochdrucklampe,
Fig. 2 einen schematischen Schnitt durch einen Klimaschrank mit der erfindungsgemässen Quecksilberdampf-Hochdrucklampe nach Fig. 1,
Fig. 3 einen Schnitt entlang Ebene S durch die erfindungsgemässe Quecksilberdampf-Hochdrucklampe nach Fig. 1 bei horizontaler Montage der Lampe,
Fig. 4 einen horizontalen Schnitt durch einen Klimaschrank mit Drehkarussell, Transportvorrichtung und horizontaler erfindungsgemässen Quecksilberdampf-Hochdrucklampe und
Fig. 5 einen horizontalen Schnitt durch einen Klimaschrank mit zwei Lagerschächten, Transportvorrichtung und horizontaler erfindungsgemässen Quecksilberdampf-Hochdrucklampe.

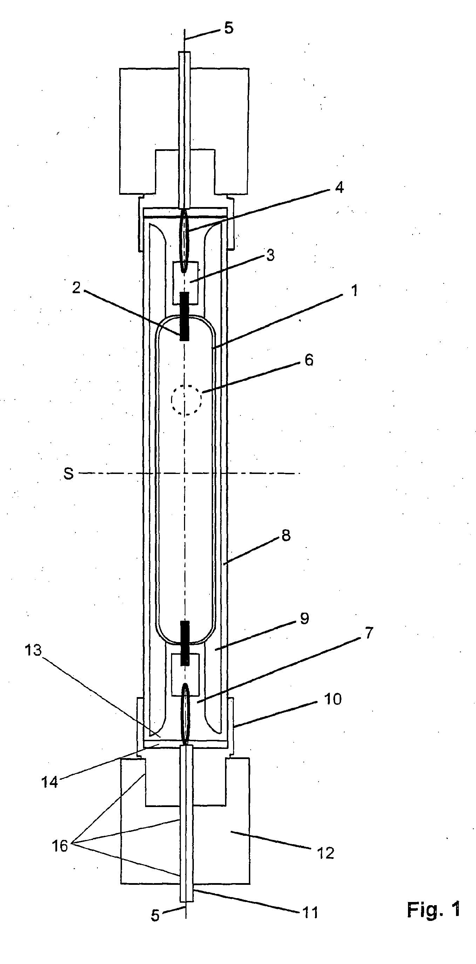

Im Folgenden wird zuerst anhand von Fig. 1 der Aufbau einer bevorzugten Ausführung der erfindungsgemässen Quecksilberdampf-Hochdrucklampe beschrieben und sodann anhand der weiteren Figuren deren Anordnung in einem Klimaschrank.

Die Quecksilberdampf-Hochdrucklampe nach Fig. 1 besteht aus einem zylindrischen, beidseitig abgeschlossenen Kolben 1 aus Quarzglas, an dessen Enden jeweils eine Metallelektrode 2 aus Wolfram in den Kolben 1 hineinragt. Die Lampe ist symmetrisch zur Symmetrieebene S ausgeführt.

Die Elektroden 2 sind über die vakuumdichten Durchführungen 3 und die Drahtleitungen 4 mit den elektrischen Zuleitungen 5 verbunden, welche zur elektrischen Stromversorgung 34 der erfindungsgemässen Quecksilberdampf-Hochdrucklampe führen. Die erforderlichen Isolationsverhältnisse werden nicht besonders beschrieben, da sie dem Fachmann bekannt sind.

Der Kolben 1 ist mit der für Quecksilberdampf-Hochdrucklampen typischen Gasfüllung 6 ausgestattet. Der Kolben 1 ist über den Stengel 7 aus Glas oder Quarzglas und über den Kragen 13 aus Glas oder Quarzglas mit dem Aussenrohr 8 vakuumdicht verschmolzen. Der Zwischenraum 9 ist mit einem Gas, vorzugsweise Stickstoff, gefüllt zur Erhöhung der Wärmeleitung zwischen Kolben 1 und Aussenrohr 8.

Alle Teile 1, 7, 8 und 13 bestehen vorzugsweise aus synthetischem Quarzglas zwecks Erzielung der physikalisch maximal möglichen UV-Transmission, da insbesondere der kürzestwellige UV-Bereich von 180 nm bis 230 nm ozonogen wirkt. Somit kann in den Nutzraum des Klimaschranks Licht mit einer Wellenlänge kleiner als 250 nm, insbesondere kleiner als 230 nm, eingestrahlt werden.

Die Ummantelung des inneren Kolbens 1 mit dem Aussenrohr 8 dient zwei Funktionen, nämlich der Erhöhung der Ozonerzeugung sowie dem Auffangen des Quecksilbers im Falle des Platzens des Kolbens 1 während des Brennens der erfindungsgemässen Quecksilberdampf-Hochdrucklampe.

Während des Brennens der erfindungsgemässen Quecksilberdampf-Hochdrucklampe erreicht die Wand des inneren Kolbens 1 im Bereich der Ebene S Temperaturen bis 1200 K. Oberhalb 800 K beginnt Ozon erheblich zu zerfallen, so dass unmittelbar in Lampennähe gebildetes Ozon wieder zerstört würde, wenn das Aussenrohr 8 den Kolben 1 nicht abschirmen würde. Das Aussenrohr 8 erreicht während des Brennens der erfindungsgemässen Quecksilberdampf-Hochdrucklampe im Bereich S nur maximal 700 K.

Eine Erhöhung der Ozonausbeute bei der erfindungsgemässen Lampe nach Fig. 1 kann erreicht werden, indem das Aussenrohr 8 mit einem UV-Sperrfilter - vorzugsweise im Wellenlängenband von 230 nm bis 280 nm - ausgestattet wird. Das oben zitierte Lehrbuch von M. Horvath, L. Bilitzky, J. Hüttner, "Ozone", Elsevier, Amsterdam 1985, lehrt auf Seite 21, Fig. 10, dass in diesem Wellenlängenband - dargestellt durch eine glockenförmige Kurve - eine Absorption vorliegt, die zur Zerstörung des durch Licht mit niedrigerer Wellenlänge erzeugten Ozons führt.

Wird nun in einer Ausführungsform der vorgeschlagenen Erfindung eine höhere Ausbeute an Ozon auf Kosten einer geringeren äusseren Ausbeute an UV-Licht gewünscht, dann wird dies erreicht durch eine Blockung des UV-Lichtes zwischen 230 nm und 280 nm bei gleichzeitiger Erhaltung der Transmission unterhalb 230 nm.

Eine derartige Filterung bzw. Blockung kann nun dadurch erreicht werden, dass mindestens eines der Rohre 1 (Innenkolben) oder 8 (Aussenrohr) mit einem entsprechenden Filter ausgestattet ist.

Solche Filter können entweder durch auf den Rohren liegenden Schichten dargestellt werden oder durch die Art des Rohrmaterials selbst.

In einer bevorzugten Ausführungsform besteht das Rohrmaterial aus synthetischem Quarzglas mit eingebetteten Nanopartikeln aus elektrisch leitenden oder auch dielektrischen Stoffen. Die theoretischen Grundlagen für derartige Filtereinrichtungen (Mie-Filter) sind beschrieben z.B. in: M. Born, E. Wolf, "Principles of Optics", Pergamon Press, Oxford 1980, 633-664.

Die Gefahr des Platzens des Kolbens 1 erhöht sich im Laufe der Betriebslebenszeit einer Quecksilberdampf-Hochdrucklampe insbesondere wegen der Rekristallisation des Quarzglasmaterials bei hohen Temperaturen. Eine gewisse Grundwahrscheinlichkeit für das Platzen existiert aber immer. Das Austreten von Quecksilberdampf in den Nutzraum würde diesen nachhaltig kontaminieren.

Im Falle des Kolbenplatzens bleibt das Quecksilber der Gasfüllung im Volumen von 8 gefangen. Die Gefahr des gleichzeitigen Berstens des Aussenrohres 8 ist um Grössenordnungen geringer, da es im Betrieb keiner Rekristallisation unterliegt und wandstärker als der Kolben 1 ist.

Der Lampenkörper, bestehend aus den Komponenten 1 bis 9 und 13, besitzt zylindrische Form und ist beidseitig mit Anschlusssockeln 10 versehen. Die Anschlusssockel 10 bestehen aus Metall, beispielsweise rostfreiem Stahl oder Leichtmetall, und sind luftdicht mit dem Quecksilberdampf-Hochdrucklampenkörper und insbesondere auch mit dem elektrischen Isolator 11 der Anschlussleitungen verkittet. Die Abdichtung gegen Luft soll die Oxidation der Durchführungen 3 bei den hohen herrschenden Temperaturen verhindern. Ferner verhindern die luftdichten Abdichtungen auch den Zutritt von Wasserdampf zu den Durchführungen 3 während der biologischen Präparation bei ausgeschalteter Lampe.

Die Anschlusssockel 10 sind über die Kontaktflächen 16 thermisch mit Kühlkörpern 12 z. B. aus Leichtmetall verbunden, welche den Betrieb der Quecksilberdampf-Hochdrucklampe in einer Umgebung mit Temperaturen bis 440 K erst ermöglichen. Die Kolbenflächen 16 sind mit Wärmeleitpaste belegt. Durch die Kühlkörper 12 wird ein höherer Wärmeleitwert zwischen Lampe und Umgebung hergestellt, so dass der notwendige Wärmetransport aus der Quecksilberdampf-Hochdruck-lampe heraus - trotz geringerer Temperaturdifferenz zwischen Lampe und Umgebung - aufrechterhalten bleibt.

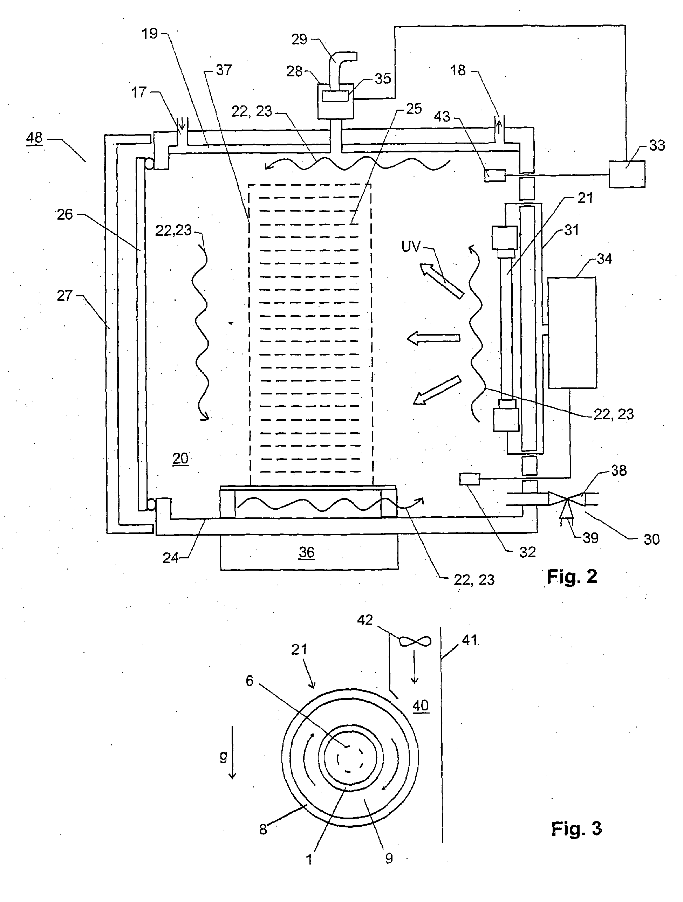

Fig. 2 zeigt schematisch einen Querschnitt durch eine Ausführung eines erfindungsgemässen Klimaschrank. Dieser besitzt eine schematisch dargestellte Klimaregelung 36, die es in bekannter Weise ermöglicht, im Nutzraum 20 des Schranks eine definierte Temperatur und ggf. definierte atmosphärische Verhältnisse (insbesondere Feuchte) bereitzustellen.

Im Nutzraum 20 sind Lagerplätze zur Aufnahme von Laborgütern vorhanden. Vorzugsweise werden diese von einem oder mehreren Lagerschächten 37 gebildet, die eine Vielzahl von seitlichen Ablagestegen 25 besitzen und so mehrere Lagerpositionen übereinander bereitstellen.

Die Lagerschächte 37 können stationär oder z.B. rotierbar auf einem Karussell gebildet sein. Im Klimaschrank kann weiter auch eine Transporteinrichtung zum automatischen Zugriff auf die Laborgüter und/oder auch eine Schüttelvorrichtung für die Proben vorgesehen sein. Entsprechende Anordnungen und Lagerschächte sind z.B. in WO 02/059251 beschrieben.

Im Nutzraum 20 zeigt Fig. 2 die Quecksilberdampf-Hochdrucklampe 21, vorzugsweise bestehend aus allen Elementen der Fig. 1, sowie die thermisch bewegten Flüsse der während des Betriebes wirkenden Medien Heissluft 22, Ozon 23 und UV-Strahlung (UV). Die Innenwände 24 des Nutzraumes 20 haben vorzugsweise die UV-Strahlung reflektierende Oberflächen, z.B. aus rostfreiem Stahlblech.

Die Laborgüter und/oder die Lagerschächte 37 können z.B. manuell durch die Front- bzw. Benutzertüren 26 und 27 ein- und ausgebracht werden. Dabei besteht die innere Fronttüre 26 teilweise aus UV-absorbierendem Glas, die äussere Fronttüre 27 hingegen aus nicht transparentem, strahlungsundurchlässigem Material, z.B. Stahlblech. Durch diese. Anordnung kann auch während des Betriebes der Quecksilberdampf-Hochdrucklampe 21 der Nutzraum 20 nach Öffnen der Türe 27 kurzzeitig zur Kontrolle eingesehen werden, wobei im übrigen die Fronttüre 27 während der Brennphase der Lampe aus Sicherheitsgründen geschlossen bleiben muss.

Eine elektronische Schaltung sorgt dafür, dass die Quecksilberdampf-Hochdruck-lampe 21 nach Ablauf einer vorgegebenen Sicherheitszeitspanne bei offener Türe 27 automatisch abgeschaltet wird. Die Fronttüre 26 wird ohnehin erst nach Verlöschen der Quecksilberdampf-Hochdrucklampe 21 und Ablauf einer weiteren Sicherheitszeitspanne entriegelt.

Als eine Sicherheitsvorrichtung gegen das Austreten von Ozon in die Laborumgebung ist eine Abluftvorrichtung 28 vorgesehen. Diese enthält eine schwache Vakuumpumpe, die den Nutzraum 20 während der Ozon erzeugenden Entkeimungsphase unter schwachem Unterdruck gegenüber der Laborumgebung hält, um das unkontrollierte Austreten von Ozon durch ungewollte Lecks zu vermeiden. Dieser Unterdruck wird mit Hilfe des Drucksensors 43 und einer Druckregelschaltung 33 überwacht, welche auf die Vakuumpumpe der Abluftvorrichtung 28 einwirkt. Der Abluftleitung 29 ist ein Katalysator 35 zur Ozonzerlegung in Normalsauerstoff vorgeschaltet.

Nach dem Abschalten der Quecksilberdampf-Hochdrucklampe 21 und während des Ablaufs einer Sicherheitszeitspanne wird über das Ventil 30, z.B. ein 3-WegeVentil, die Vorluftleitung 39 geöffnet, so dass das im Nutzraum 20 vorhandene Ozon über die Abluftvorrichtung 28 rascher ausgespült werden kann. Erst nach dieser Zeitspanne kann die innere Fronttür 26 geöffnet werden.

Die Vorluftleitung kann auch dazu verwendet werden, dem Nutzraum während der Entkeimung Sauerstoff zuzuführen, wodurch eine höhere Ozonkonzentration erreicht werden kann.

Während der Entkeimungsphase wird die Temperatur im Nutzraum 20 über die elektrische Leistung der Quecksilberdampf-Hochdrucklampe 21 geregelt. Hierzu ist ein Temperaturfühler 32 im Nutzraum 20 angeordnet, dessen Signal einer Regelschaltung in der Lampenstromversorgung 34 zugeführt wird. Die Regelschaltung steuert die über die Zuleitungen 31 an die Quecksilberdampf-Hochdrucklampe 21 abgegebene Versorgungsleistung so, dass im Nutzraum 21 ein vorgegebenes Temperaturintervall eingehalten wird.

An sich ist eine möglichst hohe Temperatur bis ca. 440 K im Nutzraum erwünscht; 440 K ist die Standard-Temperatur bei Heissluftsterilisation. Diese Temperatur ist in der Laborpraxis nicht immer erreichbar, weil sich andere, nicht in Fig. 2 dargestellt, besondere Vorrichtungen im Nutzraum 20 befinden. In diesem Falle wird der Klimaschrank so eingestellt, dass während des Entkeimungsvorgangs das sachbedingte vorgegebene Temperaturintervall im Nutzraum 20 eingehalten wird. Es versteht sich von selbst, dass die Aufgabe der Entkeimung umso mehr auf die Ozonbegasung und die UV-Bestrahlung verlagert wird, je niedriger die Lufttemperatur im Nutzraum ist. Je niedriger dabei die Lufttemperatur ist, desto höher ist auch die erreichbare maximale Ozonkonzentration, da Ozon mit zunehmender Temperatur zerfällt.

Um eine hohe UV-Bestrahlung und eine starke Ozon-Begasung auch bei Temperaturbeschränkung zu erreichen, ist, wie in Fig. 2 beispielhaft dargestellt, die obere Innenwand 24 des Nutzraumes 20 als Kühlwand 19 ausgebildet. Die Kühlwand 19 besitzt einen Hohlraum, der von einem kühlenden Fluid, zum Beispiel Luft oder Wasser, vom Zufluss 17 bis zum Ausfluss 18 durchströmt wird. Durch Voreinstellung eines bestimmten Fluid-Flusses kann eine gewisse Wärmegrundlast abgeführt werden. Die Feinregulierung der Temperatur im Nutzraum übernimmt dann beispielsweise die Regelschaltung in der Lampenstromversorgung 34. Alternativ kann auch bei konstantem Lampenstrom das im Nutzraum 20 gewünschte Temperaturintervall durch die Regelung des Fluid-Flusses eingehalten werden. Das Fluid kann auch mit einer Kältemaschine gekühlt werden.

In Fig. 2 ist eine Ausführung des Klimaschranks dargestellt, in welcher die Quecksilberdampf-Hochdrucklampe 21 in senkrechter Einbaulage, d.h. mit der Längsachse in Fallrichtung, eingesetzt wird. Diese Einbaulage hat bestimmte Vorteile, sowohl für die Lampe selbst als auch für den Entkeimungsvorgang.

Der aus Quarzglas bestehende Kolben 1 der Quecksilberdampf-Hochdrucklampe unterliegt im Betrieb sehr hohen Temperaturen, bei denen eine schwache Erweichung der Kolbenwand eintritt, die bei waagrechter Einbaulage zu einem Durchbiegen führen kann.

Im Kolben 1 kann sich die thermische Bewegung der Gasfüllung 6 bzw. des daraus entstehenden Plasmas in bekannter vorteilhafter Weise entwickeln. Ein ähnlicher Vorgang spielt sich innerhalb des Füllgases im Zwischenraum 9 ab; es ergeben sich thermisch stabile Bedingungen für den Wärmetransport.

Die gleiche Physik gilt auch für den Nutzraum 20. Längs der Quecksilberdampf-Hochdrucklampe 21 bildet sich eine Aufwärtsströmung der Luft aus, die sich insbesondere an den Aussenkolben 8 und den Kühlkörpern 12 erwärmt.

Die senkrechte Einbaulage sorgt für eine gleichmässige Verteilung der Heissluft sowie des in der Nähe der Quecksilberdampf-Hochdruck-lampe erzeugten Ozons. Obwohl das UV-Licht keiner Thermik unterliegt, so ist doch eine zentrale, senkrechte Position im Hinblick auf die meisten praktischen Einbauten die Günstigere.

Ist im Falle besonderer Innenbauten im Nutzraum eine waagrechte Einbaulage erforderlich, so sollte im Nutzraum eine mechanische Umluftförderung vorgesehen sein, und zwar vorzugsweise so, dass die erfindungsgemässe Quecksilberdampf-Hochdrucklampe 21 bezüglich ihrer Längsachse asymmetrisch gekühlt wird.

Fig. 3 erläutert diesen Gedanken. Gezeigt ist ein Querschnitt der Quecksilberdampf-Hochdrucklampe aus Fig. 1 in der Ebene S mit den gleichen Bezeichnungen, ferner ein Luftkanal 40 mit einer von der Umluftförderung 42 erzwungenen Luftströmung 41. Der Pfeil g weist in Fallrichtung.

Wesentlich ist nun, dass nur eine der in Fallrichtung g abfallenden (d.h. vertikalen) Seiten des Rohres 8 angeströmt und somit gekühlt wird, wodurch diese im Betrieb wesentlich kälter als die gegenüberliegende Seite ist. Dann bildet sich eine zirkuläre thermische Bewegung um den Kolben 1 im Zwischenraum 9 aus. Diese Bewegung führt zu stabilen thermischen Bedingungen für den Kolben 1.

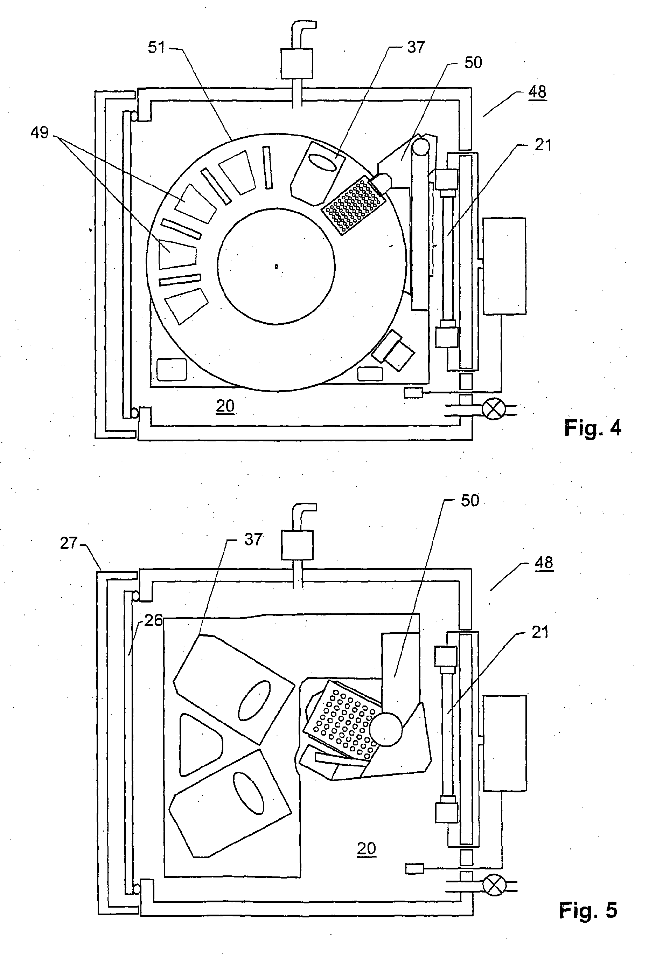

Fig. 4 zeigt einen horizontalen Schnitt durch einen Klimaschrank 48 mit horizontal angeordneter Quecksilberdampf-Hochdrucklampe 21. In dieser Ausführung sind die Lagerschächte 37 (von denen in Fig. 4 nur einer dargestellt ist) auf einem drehbaren Karussell 51 angeordnet und können von einer automatischen Transportvorrichtung 50 zugegriffen werden. Durch Öffnungen 49 im Karussell 51 wird eine Schattenbildung vermieden und die UV-Strahlung kann sich gleichmässig verteilen.

Die Verwendung der Quecksilberdampf-Hochdrucklampe 21 innerhalb eines solchen Nutzraumes 20, ist insbesondere vorteilhaft bei automatisierten Klimaschränken 48, beispielsweise mit Transportvorrichtung 50 und/oder Karussell 51. Nach dem Stand der Technik müssen Transportvorrichtung 50 und das Karussell 51 zum Zwecke der Entkeimung ausgebaut werden. Die nach dem Stand der Technik angewendete in-situ HeissluftSterilisation vermag der erfindungsgemässen Aufgabe, nämlich der Entkeimung eines möglichst breiten Spektrums aus der Vielzahl denkbarer Keime nicht zu entsprechen, insbesondere weil die Standard-Temperatur von 440 K für Heissluftentkeimung bei weitem nicht erreicht wird.

Fig. 5 zeigt ebenfalls einen horizontalen Schnitt durch einen Klimaschrank mit horizontal angeordneter Quecksilberdampf-Hochdrucklampe 21. In dieser Ausführung sind genau zwei V-förmig angeordnete Lagerschächte 37 vorgesehen. Wiederum besitzt der Klimaschrank eine Transportvorrichtung 50, welche automatisch auf die Laborgüter in den Lagerschächten 37 zugreifen kann. Die Verwendung der Quecksilberdampf-Hochdrucklampe 21 in einem solch automatisierten Klimaschrank ist aus den gleichen Gründen besonders vorteilhaft, wie bereits bei Fig. 4 dargelegt wurde.

Wenn sich im Nutzraum bewegliche Teile befinden (wie z.B. das Karussell von Fig. 4), so können diese während der Entkeimung automatisch bewegt werden, um so die Homogenität der UV-Bestrahlung zu verbessern.

## Patentansprüche

1. Einrichtung, insbesondere Klimaschrank, mit Nutzraum (20) zur kontrollierten Aufbewahrung und/oder Präparation biologischer Proben und/oder Güter, **dadurch gekennzeichnet, dass** die Einrichtung eine Quecksilberdampf-Hochdrucklampe (21) aufweist zum Entkeimen des Nutzraums, vor der Aufnahme der Proben und/oder Güter.

2. Einrichtung nach Anspruch 1, wobei ein Kolben (1) der Quecksilberdampf-Hochdrucklampe (21) von einem für UV-Strahlung durchlässigen Aussenrohr (8), umhüllt ist derart, dass nach Bersten des Kolbens (1) kein Quecksilber in den Nutzraum (20) gelangt.

3. Einrichtung nach Anspruch 2, wobei bei der Quecksilberdampf-Hochdrucklampe (21) das Aussenrohr (8) über einen Kragen (13) mit einem Stengel (7) vakuumdicht verschmolzen ist, und insbesondere wobei der Stengel (7) mit dem Kolben (1) verbunden ist.

4. Einrichtung nach Anspruch 3, wobei im Stengel (7) mindestens eine elektrische Durchführung (3, 4) zu Elektroden (2) eingebettet ist.

5. Einrichtung nach einem der Ansprüche 3 oder 4, wobei zwischen dem Aussenrohr (8) und dem Kolben (1) ein gasdichter Zwischenraum (9) besteht, der mit einem Gas gefüllt ist, das keinen Sauerstoff enthält, vorzugsweise Stickstoff.

6. Einrichtung nach einem der vorangehenden Ansprüche mit mindestens einem gasdicht mit der Quecksilberdampf-Hochkdrucklampe (21) verbundenen Anschlusssockel (10), wobei ein Isolator (11) der elektrischen Zuleitung (5) luftdicht mit dem Anschlusssockel (10) verbunden ist.

7. Einrichtung nach Anspruch 6, wobei in einem Hohlraum (14) zwischen der Quecksilberdampf-Hochkdrucklampe (21) und dem Anschlusssockel (10) Wärmeleitpaste angeordnet ist.

8. Einrichtung nach einem der vorangehenden Ansprüche, wobei an der Quecksilberdampf-Hochdrucklampe (21) mindestens ein Kühlkörper (12) angeordnet ist, wobei der Kühlkörper (12) möglichst formschlüssig angebracht ist und die Kontaktflächen (16) vorzugsweise mit Wärmeleitpaste belegt werden.

9. Einrichtung nach einem der vorangehenden Ansprüche, wobei sie derart ausgestaltet ist, dass die Temperatur an einer Oberfläche des Aussenrohrs (8) bei Betrieb der Quecksilberdampf-Hochdrucklampe (21) höchstens 700 K beträgt.

10. Einrichtung nach einem der vorangehenden Ansprüche, wobei die Quecksilberdampf-Hochdrucklampe (21) unmittelbar im Nutzraum (20) derart angebracht ist, dass eine direkte oder über reflektierende Flächen indirekte Sichtverbindung zur gleichmässigen Verteilung der UV-Strahlung zu den mechanischen Strukturen im Nutzraum besteht.

11. Einrichtung nach Anspruch 10, wobei Innenwände (24) des Nutzraumes (20) die UV-Strahlung reflektierende Oberflächen besitzen.

12. Einrichtung nach einem der vorangehenden Ansprüche, wobei der Nutzraum (20) luftdicht/aerosoldicht gegen die äussere Atmosphäre ausgeführt ist.

13. Einrichtung nach einem der vorangehenden Ansprüche mit einer inneren Benutzertüre (26) bestehend mindestens teilweise aus UV-undurchlässigem Glas und einer äusseren, nicht transparenten Benutzertüre (27).

14. Einrichtung nach einem der vorangehenden Ansprüche mit einer Abluftvorrichtung (28) zum Erzeugen eines Unterdrucks im Nutzraum (20), wobei insbesondere in der Abluftvorrichtung (28) ein Katalysator (35) zum Ozonabbau angeordnet ist.

15. Einrichtung nach Anspruch 14 mit einer Luftzuleitung (39) und einem Ventil (30) zum Öffnen bzw. Schliessen der Luftzuleitung (39), und insbesondere wobei die Einrichtung ausgestaltet ist um nach Abschalten der Lampe (21) und vor Öffnen einer Türe (26) in einer Entkeimungsphase im Nutzraum (20) befindliches Ozon durch Einlass von Luft über die Luftleitung (39) und das Ventil (30), insbesondere ein 3-WegeVentil, und über die Abluftvorrichtung (28) abzubauen und auszuspülen.

16. Einrichtung nach Anspruch 15, derart ausgestaltet, dass während der Entkeimungsphase die Ozonkonzentration im Nutzraum (20) durch Einlass von Sauerstoff über die Zuleitung (38) und das Ventil (30) erhöht wird derart, dass im Nutzraum (20) der eingestellte Unterdruck vermöge der Abluftvorrichtung (28) und der Drucküberwachung (43) aufrecht erhalten wird.

17. Einrichtung nach einem der vorangehenden Ansprüche, derart ausgestaltet, dass während der Entkeimung die Lage von im Nutzraum befindlichen, beweglichen mechanischen Elementen in der Weise verändert wird, dass eine möglichst gleichmässige UV-Bestrahlung erfolgt.

18. Einrichtung insbesondere nach einem der vorangehenden Ansprüche, wobei die Quecksilberdampf-Hochdrucklampe (21) eine vertikal angeordnete Längsachse besitzt.

19. Einrichtung nach einem der vorangehenden Ansprüche, wobei die Quecksilberdampf-Hochdrucklampe (21) eine horizontal angeordnete Längsachse besitzt, wobei die Quecksilberdampf-Hochdrucklampe (21) asymmetrisch gekühlt ist, und insbesondere wobei eine Umluftförderung (42) vorgesehen ist, mit welcher eine erzwungene Luftströmung (41) an nur eine vertikale Seite der Lampe (21) heranführbar ist, derart, dass im einem Zwischenraum (9) der Lampe (21) eine thermische Gasbewegung entsteht.
die Quecksilbderdampf-Hochdrucklampe (21) horizontal mittig an einer Innenwand des Nutzraums (20) angeordnet ist, insbesondere gegenüber einer Benutzertüre (26, 27) der Einrichtung.

20. Einrichtung nach einem der vorangehenden Ansprüche, wobei mit der Quecksilberdampf-Hochdrucklampe (21) in den Nutzraum (20) Licht mit einer Wellenlänge kleiner als 250 nm, insbesondere kleiner als 230 nm, einstrahlbar ist.

21. Einrichtung nach einem der vorangehenden Ansprüche mit einer Regelschaltung (34), mit welcher über eine an die Quecksilberdampf-Hochdrucklampe (21) zugeführte Versorgungsleistung die Temperatur im Nutzraum (20) regelbar ist.

22. Einrichtung nach einem der vorangehenden Ansprüche, wobei der Nutzraum (20) mit einer vorzugsweise von einem Fluid durchströmten Kühlwand (19) versehen ist derart, dass die Temperatur im Nutzraum (20) während des Brennens der Quecksilberdampf-Hochdrucklampe (21) unabhängig von der Lampenleistung beschränkt und/oder geregelt werden kann.

23. Klimaschrank nach einem der vorangehenden Ansprüche mit einem Nutzraum (20) zur kontrollierten Aufbewahrung und/oder Präparation biologischer Proben, **dadurch gekennzeichnet, dass** im Nutzraum (20) zum Entkeimen des Nutzraums eine Quecksilberdampf-Hochdrucklampe (21) angeordnet ist.

24. Klimaschrank nach einem der vorangehenden Ansprüche, wobei mit der Quecksilberdampf-Hochdrucklampe (21) in den Nutzraum (20) Licht mit einer Wellenlänge kleiner als 250 nm, insbesondere kleiner als 230 nm, einstrahlbar ist.

25. Verfahren zur Entkeimung von Nutzräumen (20) zur Aufbewahrung und/oder Präparation von biologischen Proben und/oder biologischer Güter, insbesondere in einem Klimaschrank (48), **dadurch gekennzeichnet, dass** der Nutzraum vom UV-Licht einer Quecksilberdampf-Hochdrucklampe (21) beaufschlagt wird.

26. Verfahren zur Entkeimung von Nutzräumen (20) zur Aufbewahrung und/oder Präparation von biologischen Proben und/oder biologischer Güter, insbesondere nach Anspruch 25, bei welchem mit einer Quecksilberdampf-Hochdrucklampe (21) in einem Nutzraum (20) Ozon erzeugt wird, wobei die Ozonkonzentration der in im Nutzraum (20) enthaltenen Luft durch permanentes Zyklieren derselben innerhalb des Nutzraums infolge der von der Quecksilberdampflampe (21) ausgehenden ozonogenen UV-Strahlung bis über die bioziden Schwellwerte erhöht wird.

27. Verfahren insbesondere nach einem der Ansprüche 25 oder 26 zur Entkeimung von Nutzräumen (20) zur Aufbewahrung und/oder Präparation von biologischen Proben und/oder biologischer Güter, insbesondere Klimaschrank (48), durch eine im luftdichten/aerosoldichten Nutzraum angebrachte erfindungsgemässe Quecksilberdampflampe (21).

28. Quecksilberdampflampe oder Einrichtung in Verbindung mit einer Quecksilberdampflampe, **dadurch gekennzeichnet, dass** ein das Licht durchlassendes Hüllrohr der Quecksilberdampflampe aus Quarzglas mit eingebetteten Nanopartikeln besteht, derart, dass das durch die Nanopartikel entstandene Blockfilter ozonogene Strahlung zwischen 180 nm und 230 nm durchlässt und ozonzerlegende Strahlung zwischen 230 nm und 280 nm unterdrückt.

29. Quecksilberdampflampe oder Einrichtung mit einer Quecksilberdampflampe, insbesondere nach einem der Ansprüche 1 bis 24 oder 28, **dadurch gekennzeichnet, dass** das Licht durchlassende Hüllrohr der Lampe mit einem Blockfilter - insbesondere aus Quarzglas mit darin eingebetteten Nanopartikeln - versehen ist, derart, dass das Blockfilter, die ozonogene Strahlung zwischen 180 nm und 230 nm durchlässt und die ozonzerlegende Strahlung zwischen 230 nm und 280 nm unterdrückt.

30. Verwendung der Quecksilberdampflampe bzw. Vorrichtung nach Anspruch 29 zum Entkeimen eines Nutzraums.

31. Einrichtung nach einem der Ansprüche 1 bis 24 oder 28 oder 29, wobei ein Aussenrohr (8) der Quecksilberdampf-Hochdrucklampe für Strahlung mit einer Wellenlänge zwischen 180 nm und 230 nm besser durchlässig ist als für Strahlung mit einer Wellenlänge zwischen 230 nm und 280 nm und/oder dass ein Aussenrohr (8) der Quecksilberdampf-Hochdrucklampe aus Quarzglas mit eingebetteten Nanopartikeln besteht.
